(19) Europäisches Patentamt
European Patent Office
Office européen des brevets

(11) **EP 3 304 035 B1**

(12) **EUROPÄISCHE PATENTSCHRIFT**

(45) Veröffentlichungstag und Bekanntmachung des Hinweises auf die Patenterteilung:
**27.11.2019 Patentblatt 2019/48**

(51) Int Cl.:
*G01N 11/04* *(2006.01)*       *G01N 11/08* *(2006.01)*

(21) Anmeldenummer: **16732919.2**

(86) Internationale Anmeldenummer:
**PCT/AT2016/050180**

(22) Anmeldetag: **07.06.2016**

(87) Internationale Veröffentlichungsnummer:
**WO 2016/197169 (15.12.2016 Gazette 2016/50)**

(54) **MESSDÜSE ZUR BESTIMMUNG DER DEHNVISKOSITÄT VON POLYMERSCHMELZEN**

MEASURING NOZZLE FOR DETERMINING THE EXTENSIONAL VISCOSITY OF POLYMER MELTS

BUSE DE MESURE POUR DÉTERMINER LA VISCOSITÉ EXTENSIONNELLE DE MATIÈRES POLYMÈRES FONDUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.06.2015 AT 504652015**

(43) Veröffentlichungstag der Anmeldung:
**11.04.2018 Patentblatt 2018/15**

(73) Patentinhaber: **Leistritz Extrusionstechnik GmbH 90459 Nürnberg (DE)**

(72) Erfinder:
• **MIETHLINGER, Jürgen**
 **4851 Gampern (AT)**
• **LÖW-BASELLI, Bernhard**
 **4320 Perg (AT)**
• **LUGER, Hans Jürgen**
 **4020 Linz (AT)**

(74) Vertreter: **Lindner Blaumeier Patent- und Rechtsanwälte Partnerschaftsgesellschaft mbB Dr. Kurt-Schumacher-Str. 23 90402 Nürnberg (DE)**

(56) Entgegenhaltungen:
**US-A- 5 357 784**

**Beschreibung**

Technisches Gebiet

Die Erfindung bezieht sich auf eine Messdüse zur Bestimmung der Dehnviskosität von Polymerschmelzen während ihrer Verarbeitung mit einem einen rechteckigen Querschnitt aufweisenden Strömungskanal, der zwischen einem Einlaufabschnitt und einem Auslaufabschnitt jeweils konstanten Querschnitts einen Übergangsabschnitt aufweist, der sich in Strömungsrichtung hyperbolisch verjüngt.

Stand der Technik

Eine Bestimmung der Dehnviskosität mithilfe von Druckfühlern, die einer Verjüngung eines Strömungskanals einer Messdüse vor- und nachgeordnet sind, setzt eine konstante mittlere Dehnrate der Polymerschmelze im Verjüngungsabschnitt des Strömungskanals voraus. Zu diesem Zweck ist es bekannt (US 5 357 784 A, US 6 220 083 B1), eine Messdüse mit einem Einlaufabschnitt und einem Auslaufabschnitt jeweils konstanten Querschnitts und mit einem den Einlaufquerschnitt auf den Auslaufquerschnitt verjüngenden Übergangsabschnitt vorzusehen, der zur Ausbildung der Verjüngung zwei einander gegenüberliegende Kanalwände mit einem hyperbolischen Verlauf, im Übrigen aber einen rechteckigen Querschnitt zwischen den konvergierenden Kanalwänden und den diese konvergierenden Kanalwände miteinander verbindenden, zueinander parallel verlaufenden Kanalwänden aufweist. Bedingt durch diese Geometrie des Übergangsabschnitts zwischen dem Einlauf- und dem Auslaufabschnitt der Messdüse kann eine weitgehend konstante mittlere Dehnungsrate für die Polymerschmelze sichergestellt werden, allerdings mit dem Nachteil eines vergleichsweise geringen Druckabfalls, was bei höheren Anforderungen an die Messgenauigkeit eine hohe Ansprechempfindlichkeit der eingesetzten Druckfühler erfordert. Abgesehen davon, dass solche Druckfühler über in die Messdüse gebohrte Messkapillaren an die Einlauf- und Auslaufabschnitte des Strömungskanals angeschlossen werden, was die Gefahr von Anlagerungen in diesen Messkapillaren mit sich bringt, können handelsübliche Druckfühler den Anforderungen an die Messgenauigkeit kaum genügen.

Darstellung der Erfindung

Der Erfindung liegt somit die Aufgabe zugrunde, eine Messdüse zur Bestimmung der Dehnviskosität von Polymerschmelzen so auszubilden, dass trotz des Einsatzes handelsüblicher Druckfühler eine ausreichende Messgenauigkeit gewährleistet werden kann.

Zur Lösung ist erfindungsgemäß eine Messdüse zur Bestimmung der Dehnviskosität von Polymerschmelzen während ihrer Verarbeitung mit einem einen rechteckigen Querschnitt aufweisenden Strömungskanal, der zwischen einem Einlaufabschnitt und einem Auslaufabschnitt jeweils konstanten Querschnitts einen Übergangsabschnitt aufweist, der sich in Strömungsrichtung verjüngt, wobei der Übergangsabschnitt eine einlaufseitige Zone, in der sich der gegenseitige Abstand zweier einander gegenüber liegender erster Kanalwände zwischen zwei einander gegenüberliegenden hyperbolischen zweiten Kanalwänden in Strömungsrichtung stetig verringert, und eine daran anschließende auslaufseitige Zone, in der die beider einander paarweise gegenüberliegenden zweiten Kanalwände zueinander parallel verlaufen, während die beiden dazwischen angeordneten ersten Kanalwände in Strömungsrichtung hyperbolisch konvergieren, vorgesehen.

Durch die Aufteilung des Übergangsabschnitts in eine einlaufseitige Zone, in der von den einander paarweise gegenüberliegenden Kanalwänden ein Paar in Strömungsrichtung hyperbolisch und unter Einhaltung der Strömungsbedingungen für eine konstante mittlere Dehnungsrate das andere Paar stetig konvergieren, und einer daran anschließenden auslaufseitigen Zone, in der ebenfalls ein Paar der einander paarweise gegenüberliegenden Kanalwände hyperbolisch konvergiert, während das andere Paar einen konstanten gegenseitigen Abstand aufweist, wird eine Verlängerung des Strömungsabschnitts ermöglicht, in dem eine konstante mittlere Dehnrate vorherrscht. Dies führt zu einer Vergrößerung des Druckverlusts, auf dessen Basis die Dehnviskosität berechnet wird, womit die konstruktiven Voraussetzungen zum Einsatz handelsüblicher Druckfühler geschaffen wird, die trotz einer mäßigen Ansprechempfindlichkeit in Verbindung mit der erfindungsgemäßen Messdüse höhere Anforderungen an die Messgenauigkeiten erfüllen können.

Die geometrischen Voraussetzungen für eine erfindungsgemäße Messdüse erlauben den unmittelbaren Anschluss handelsüblicher Druckfühler zumindest an den einlaufseitigen Abschnitt des Strömungskanals über ein Anschlussgewinde. Begrenzen die parallelen Kanalwände der auslaufseitigen Zone des Übergangsabschnitts die Breite des Strömungskanals, sodass sich nach der Verjüngung der Kanalbreite in der einlaufseitigen Zone keine Änderung der Breite des Strömungskanals mehr ergibt, so können auch im auslaufseitigen Abschnitt der Messdüse unter Vermeidung von Messkapillaren handelsübliche Druckfühler unmittelbar an den Strömungskanal angeschlossen werden.

Kurze Beschreibung der Zeichnung

In der Zeichnung ist der Erfindungsgegenstand beispielsweise dargestellt. Es zeigen

Fig. 1     eine erfindungsgemäße Messdüse zur Bestimmung der Dehnviskosität von Polymerschmelzen in einer schematischen, zum Teil aufgerissenen Draufsicht,

Fig. 2    diese Messdüse in einem Schnitt nach der Linie II-II der Fig. 1 und

Fig. 3    den Übergangsabschnitt zwischen dem Einlauf- und dem Auslaufabschnitt der Messdüse in einem Längsschnitt in einem größeren Maßstab.

Weg zur Ausführung der Erfindung

Die dargestellte Messdüse bildet einen Strömungskanal für eine Polymerschmelze, der einen beispielsweise an einen Extruder anschließbaren Einlaufabschnitt 1 und einen Auslaufabschnitt 2 sowie einen Übergangsabschnitt 3 zwischen dem Einlauf- und Auslaufabschnitt 1, 2 umfasst. Der Strömungsquerschnitt ist über die Düsenlänge durchgehend rechteckig. Im Übergangsabschnitt 3 wird der Strömungsquerschnitt des Einlaufabschnitts 1 auf den gegenüber dem Einlaufabschnitt 1 sowohl in der Breite als auch in der Höhe verringerten Querschnitt des Auslaufabschnitts 2 verringert, und zwar unter Strömungsbedingungen, die eine konstante mittlere Dehnungsrate im Übergangsabschnitt 3 sicherstellen. Zu diesem Zweck ist der Übergangsabschnitt 3 in eine einlaufseitige Zone 4 und eine auslaufseitige Zone 5 mit jeweils unterschiedlicher geometrischer Form unterteilt. In der einlaufseitigen Zone 4 konvergieren zwei Kanalwände 6, der einander paarweise gegenüberliegenden Kanalwände 6, 7 hyperbolisch, während der gegenseitige Abstand der Kanalwände 7 des anderen Kanalwandpaars in Strömungsrichtung 8 stetig, vorzugsweise linear, abnimmt. Um in der einlaufseitigen Zone 4 eine konstante mittlere Dehnungsrate zu erhalten, muss die Breite y eines Rechteckquerschnitts an der Stelle x in Düsenlängsrichtung der Bedingung

$$y = C/(a + k_1 \cdot x) \cdot z$$

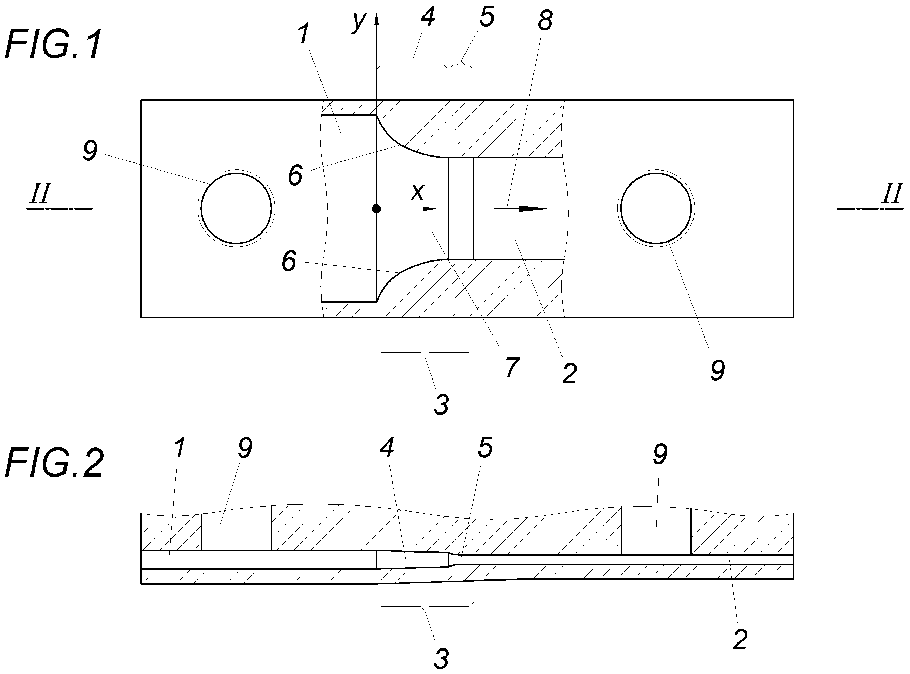

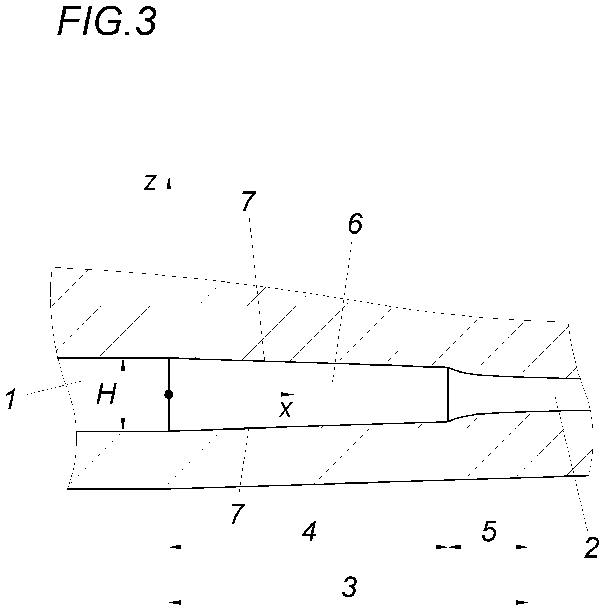

genügen, wobei C, a und $k_1$ von Strömungsbedingungen abhängigen Konstanten darstellen und z die halbe Höhe des Querschnitts an der Stelle x bedeutet. Bei einer linearen Abnahme der Höhe ergibt sich $z = H/2 - k_2 \, x$, wenn von einer mittigen x-Achse und einer Höhe H des Querschnitts des einlaufseitigen Abschnitts 1 und einer Steigung $k_2$ für die Neigung der betreffenden Kanalwand 7 gegenüber der Düsenachse ausgegangen wird, wie dies in den Fig. 1 und 3 angedeutet ist.

In der anschließenden, auslaufseitigen Zone 5 wird zur Aufrechterhaltung einer konstanten mittleren Dehnungsrate ein Paar der Kanalwände 6, 7 parallel geführt, während das andere Paar nach einer hyperbolischen Funktion in Strömungsrichtung 8 konvergiert. Im Ausführungsbeispiel konvergieren die die Höhe des Strömungskanals bestimmenden Kanalwände 7, sodass sich durch diese Kanalverjüngung im Bereich der Zone 5 keine Verringerung der Kanalbreite ergibt. Dieser Umstand erlaubt es, handelsübliche Druckfühler nicht nur im Bereich des Einlaufabschnitts 1, sondern auch im Bereich des Auslaufabschnitts 2 unmittelbar an den Strömungskanal anzuschließen. Im Ausführungsbeispiel wird dies durch Anschlussbohrungen 9 angedeutet.

Aufgrund der besonderen geometrischen Form der Messdüse wird im Vergleich zu bekannten Messdüsen der Längenbereich des Übergangsabschnitts 3 vergrößert, in dem eine konstante mittlere Dehnungsrate als Voraussetzung für die Bestimmung der Dehnviskosität von Polymerschmelzen aufrechterhalten werden kann. Die damit einhergehende Vergrößerung des Druckabfalls macht die Messdüse empfindlicher, sodass auch mit handelsüblichen Druckfühlern ausreichend genaue Messergebnisse erhalten werden können.

**Patentansprüche**

1. Messdüse zur Bestimmung der Dehnviskosität von Polymerschmelzen während ihrer Verarbeitung mit einem einen rechteckigen Querschnitt aufweisenden Strömungskanal, der zwischen einem Einlaufabschnitt (1) und einem Auslaufabschnitt (2) jeweils konstanten Querschnitts einen Übergangsabschnitt (3) aufweist, der sich in Strömungsrichtung (8) verjüngt, wobei der Übergangsabschnitt (3) eine einlaufseitige Zone (4), in der sich der gegenseitige Abstand zweier einander gegenüber liegender erster Kanalwände (7) zwischen zwei einander gegenüberliegenden hyperbolischen zweiten Kanalwänden (6) in Strömungsrichtung (8) stetig verringert, und eine daran anschließende auslaufseitige Zone (5), in der die beider einander paarweise gegenüberliegenden zweiten Kanalwände (6) zueinander parallel verlaufen, während die beiden dazwischen angeordneten ersten Kanalwände (7) in Strömungsrichtung (8) hyperbolisch konvergieren, umfasst.

2. Messdüse nach Anspruch 1, **dadurch gekennzeichnet, dass** die parallelen Kanalwände (6) der auslaufseitigen Zone (5) des Übergangsabschnitts (3) die Breite des Strömungskanals begrenzen.

**Claims**

1. Measurement nozzle for determining the extensional viscosity of polymer melts during the processing thereof, having a flow channel which has a rectangular cross section and which has, between an inlet section (1) and an outlet section (2) of in each case constant cross section, a crossover section (3) which tapers in the flow direction (8), wherein the crossover section (3) comprises an inlet-side zone (4), in which the mutual spacing between two mutually opposite first channel walls (7) between two mutually opposite hyperbolic second channel walls (6) decreases steadily in the flow direction (8), and an adjoining outlet-side zone (5), in which the two pairwise mutually opposite second channel walls (6) extend parallel to one another while the two first channel walls (7) arranged therebetween converge hyperbolically in the flow direction (8).

2. Measurement nozzle according to Claim 1, **characterized in that** the parallel channel walls (6) of the outlet-side zone (5) of the crossover section (3) delimit the width of the flow channel.

## Revendications

1. Buse de mesure destinée à déterminer la viscosité extensionnelle de polymères fondus pendant sa transformation, comprenant un canal d'écoulement qui possède une section transversale rectangulaire, lequel possède une portion de transition (3) entre une portion d'entrée (1) et une portion de sortie (2) ayant respectivement une section transversale constante, laquelle se rétrécit dans la direction de l'écoulement (8), la portion de transition (3) comportant une zone côté entrée (4), dans laquelle l'écart mutuel de deux premières parois de canal (7) situées en face l'une de l'autre, entre deux deuxièmes parois de canal (6) hyperboliques en face l'une de l'autre, se réduit continuellement dans la direction de l'écoulement (8), et une zone côté sortie (5) rattachée à celle-ci, dans laquelle les deux deuxièmes parois de canal (6) situées en face l'une de l'autre par paire s'étendent parallèlement l'une à l'autre, alors que les deux premières parois de canal (7) disposées entre celles-ci convergent de manière hyperbolique dans la direction de l'écoulement (8).

2. Buse de mesure selon la revendication 1, **caractérisée en ce que** les parois de canal (6) parallèles de la zone côté sortie (5) de la portion de transition (3) délimitent la largeur du canal d'écoulement.

FIG.1

FIG.2

FIG.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5357784 A
- US 6220083 B1